# EUROPEAN PATENT APPLICATION

(11) **EP 2 959 978 A1**
(43) Date of publication of application: **30.12.2015**
(21) Application number: 15151944.4
(22) Date of filing: 21.01.2015
(51) Int. Cl.: B05B 17/00

(54) **Spraying device and spraying module**

(30) Priority: 25.06.2014 TW 103121861
(71) Applicant: DelBio Inc., Taoyuan County, Taiwan 33341 (TW)
(72) Inventor: Lee, Yu-Wei, 33341 Taoyuan City (TW); Lee, Kuo-Liang, 33341 Taoyuan City (TW); Huang, Ren-De, 33341 Taoyuan City (TW); Lin, Chia-Ching, 33341 Taoyuan City (TW); Chang, Chih-Ching, 33341 Taoyuan City (TW)
(74) Representative: Zimmermann, Tankred Klaus

(57) **Abstract**

A spraying module includes a spraying element, a first covering element and a second covering element. The spraying element includes a first electrode and a second electrode. The first covering element has two depressions and is disposed on one side of the spraying element, and the first electrode and the second electrode are disposed in the two depressions respectively. The second covering element includes two protrusions and is disposed on the other side of the spraying element, and the two protrusions cover the first electrode and the second electrode respectively. The first covering element and the second covering element are made by compliant material.

## Description

### BACKGROUND OF THE INVENTION

### Field of Invention

The invention relates to a spraying device and, in particular, to a spraying module of a spraying device.

### Related Art

Recently, various atomization devices, i.e. spraying devices, have been largely applied to the medical or cosmetic purpose. After atomized into fine particles or drops by the spraying device, the medicinal, cosmetic or essential liquid can more easily go into the body for the health care, treatment for respiratory diseases, or making the skin absorb medicinal liquid or cosmetic liquid more rapidly, or intensifying the fragrance.

The spraying device has a space for storing the liquid and includes an oscillation element for vaporizing liquid and spraying it out. The liquid stored in the space can be gathered on the oscillation element and atomized by the oscillation element and then sprayed out. Generally, the oscillation element is electrically connected to a circuit board and driven by a piezoelectric element to generate oscillation with a frequency for atomizing the liquid.

The oscillation element must be electrically connected to the circuit board. In other words, the oscillation element must include the structure such as electrodes for the electrical connection. However, the electrode may be short-circuited due to contact with liquid or due to damping, and such that even the oscillation element may be totally damaged. Besides, since the piezoelectric element as the main body of the oscillation element is really weak or brittle in material property, an additional element to protect the piezoelectric element is required.

Although the industry has used wrapping injection method to form a flexible element at the periphery of the oscillation element to protect the oscillation element, which includes the piezoelectric element, from being damaged by hit, the piezoelectric element still could be damaged due to the mold structure when drawn out during the wrapping injection process, and therefore the production cost will be increased a lot.

Therefore, it is an important subject to provide a spraying module and a spraying device which have innovative structure design so as to prevent the oscillation element and the piezoelectric element therein from being damaged due to external force and to prevent the electrode of the spraying module from being damped.

### SUMMARY OF THE INVENTION

In view of the foregoing subject, an objective of the invention is to provide a spraying module and a spraying device which have innovative structure design so as to prevent the oscillation element and the piezoelectric element therein from being damaged due to external force and to prevent the electrode of the spraying module from being damped.

To achieve the above objective, a spraying module according to the invention comprises a spraying element, a first covering element and a second covering element. The spraying element includes a first electrode and a second electrode. The first covering element has two depressions and is disposed on one side of the spraying element. The first electrode and the second electrode are disposed in the two depressions, respectively. The second covering element has two protrusions and is disposed on the other side of the spraying element. The two protrusions cover the first electrode and the second electrode, respectively, and the first covering element and the second covering element are made by compliant material.

To achieve the above objective, a spraying device according to the invention comprises a main body, a spraying module and an output structure. The main body includes an accommodating space. The spraying module is disposed at the accommodating space and includes a spraying element, a first covering element and a second covering element. The spraying element includes a first electrode and a second electrode. The first covering element has two depressions and is disposed on one side of the spraying element. The first electrode and the second electrode are disposed in the two depressions, respectively. The second covering element has two protrusions and is disposed on the other side of the spraying element. The two protrusions cover the first electrode and the second electrode, respectively, and the first covering element and the second covering element are made by compliant material. The output structure has an output channel disposed corresponding to the spraying module.

In one embodiment, the compliant material includes silica gel, rubber, soft polymer or foam.

In one embodiment, the soft polymer includes PORON® (micro-cell polymer material), EVA (ethylene-vinyl acetate), CR (chloroprene rubber), SBR (styrene-butadiene rubber), PU (polyurethane) or PE (polyethylene).

In one embodiment, the spraying element includes a piezoelectric element.

In one embodiment, the spraying element further includes a mesh element.

In one embodiment, the first covering element has an input opening and the second covering element has an output opening.

In one embodiment, the main body has an opening at the accommodating space to communicate with an inner portion and an outer portion of the main body, and the opening is disposed corresponding to the input opening.

In one embodiment, the first covering element includes a first engaging structure, the second covering element includes a second engaging structure, and the first engaging structure and the second engaging structure match each other.

In one embodiment, the first covering element has two holes disposed corresponding to the two depressions, respectively, and the first electrode and second electrode of the spraying element are disposed corresponding to the two holes, respectively.

In one embodiment, the main body further includes a pair of third electrodes which are disposed in the accommodating space and corresponding to the first electrode and the second electrode respectively.

As mentioned above, in the spraying device and the spraying module of the invention, the first and second covering elements are disposed on the two sides of the spraying element respectively and made by compliant material with softness and elasticity, so the spraying element, especially for the weaker or brittle element of the spraying element, such as the piezoelectric element, can be protected by the disposition and material property of the first and second covering elements. The first and second covering elements not only can protect the weaker and brittle piezoelectric element from being damaged due to external force, but also can buffer the oscillation extent of the piezoelectric element supplied with electricity so that the piezoelectric element can be prevented from being damaged due to the overmuch oscillation extent. Besides, by the use of the compliant material, the first and second covering elements can have better sealing effect so that the first, second and third electrodes can be prevented from being damped and the lifespan of the spaying device and module can be thus extended.

Furthermore, the first covering element includes the depressions and the second covering element correspondingly includes the protrusions to accommodate and together cover the piezoelectric element, the first, second, and third electrodes. Therefore, the first, second and third electrodes can be enhanced in the sealed effect and can be further prevented from the damp.

### BRIEF DESCRIPTION OF THE DRAWINGS

The invention will become more fully understood from the detailed description and accompanying drawings, which are given for illustration only, and thus are not limitative of the present invention, and wherein:
FIG. 1A is a schematic diagram of a spraying device of an embodiment of the invention;
FIG. 1B is a schematic exploded diagram of the spraying device in FIG. 1A;
FIG. 2 is a schematic enlarged diagram of the spraying module in FIG. 1B;
FIG. 3A is a schematic assembled diagram of the spraying module in FIG. 2;
FIG. 3B is a schematic sectional diagram of the spraying module taken along the line A-A in FIG. 3A; and
FIG. 4 is a schematic diagram showing the first covering element disposed at the accommodating space in FIG. 1B.

### DETAILED DESCRIPTION OF THE INVENTION

The present invention will be apparent from the following detailed description, which proceeds with reference to the accompanying drawings, wherein the same references relate to the same elements.

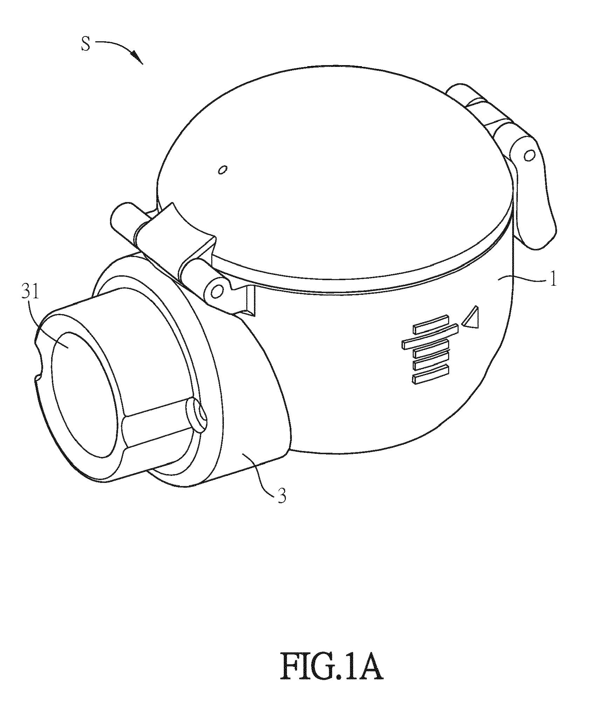

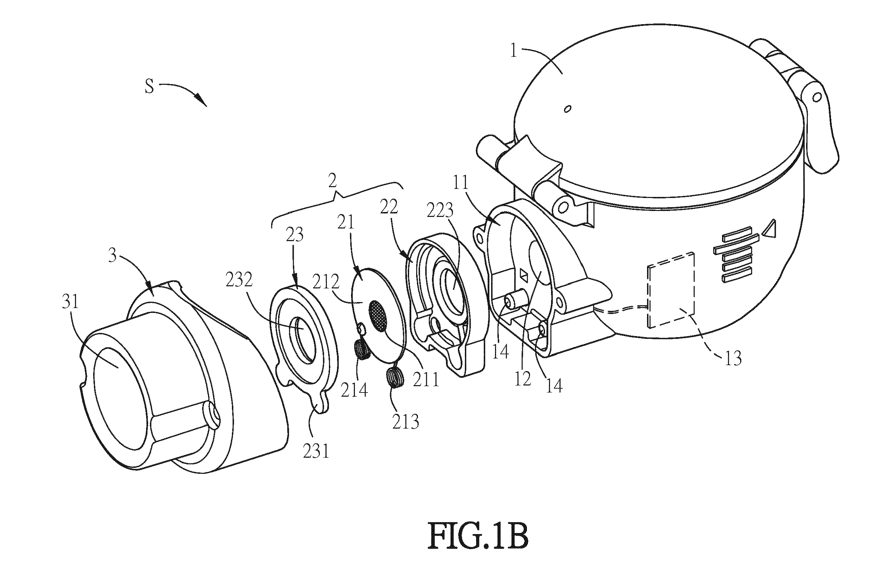

FIG. 1A is a schematic diagram of a spraying device of an embodiment of the invention, and FIG. 1B is a schematic exploded diagram of the spraying device in FIG. 1A. As shown in FIGS. 1A and 1B, the spraying device S of this embodiment includes a main body 1, a spraying module 2 and an output structure 3. The main body 1 has a liquid-store space therein for storing some kind of liquid such as medicinal, cosmetic or essential liquid. The outer side of the main body 1 has an accommodating space 11 at which the spraying module 2 can be disposed. The main body 1 has an opening 12 at the accommodating space 11 to communicate with inner portion and outer portion of the main body 1 so that the liquid stored in the inner portion of the main body 1 can be transmitted to the spraying module 2 through the opening 12.

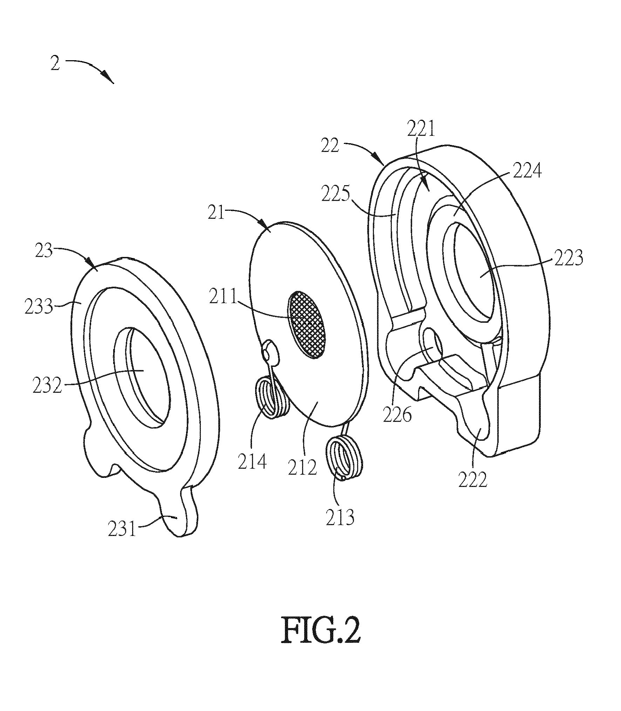

FIG. 2 is a schematic enlarged diagram of the spraying module in FIG. 1B. As shown in FIG. 2, the spraying module 2 includes a spraying element 21, a first covering element 22 and a second covering element 23. In this embodiment, the spraying element 21 is a circular sheet or disk-shaped structure and includes a mesh element 211 and a piezoelectric element 212 which are connected to each other. The piezoelectric element 212 is, for example but not limited to, PZT, Lead Zirconate Titantate. The piezoelectric element 212 can convert electric energy into mechanical energy by the piezoelectric effect. In other words, the piezoelectric element 212 undergoes an electric field effect to have a mechanical deformation. In this embodiment, the spraying element 21 includes a first electrode 213 and a second electrode 214, which are, for example but not limited to, metal coils and electrically connected to the piezoelectric element 212. The main body 1 further includes a circuit control board 13, which is electrically connected to the first and second electrodes 213 and 214 to supply the electric field (voltage) to the piezoelectric element 212 and thus can control the oscillation frequency of the piezoelectric element 212. In an embodiment, the circuit control board 13 can be disposed in the inner portion of the main body 1 and surely needs to be separated from the liquid stored in the inner portion of the main body 1. A pair of third electrodes 14, 14 are disposed in the accommodating space 11 of the main body 1 and electrically connected to the circuit control board 13 through an extended circuit of the circuit control board 13. When the spraying module 2 is disposed at the accommodating space 11, the circuit control board 13 can be electrically connected to the first and second electrodes 213 and 214 through the two third electrodes 14, 14 respectively to further control the oscillation of the piezoelectric element 212.

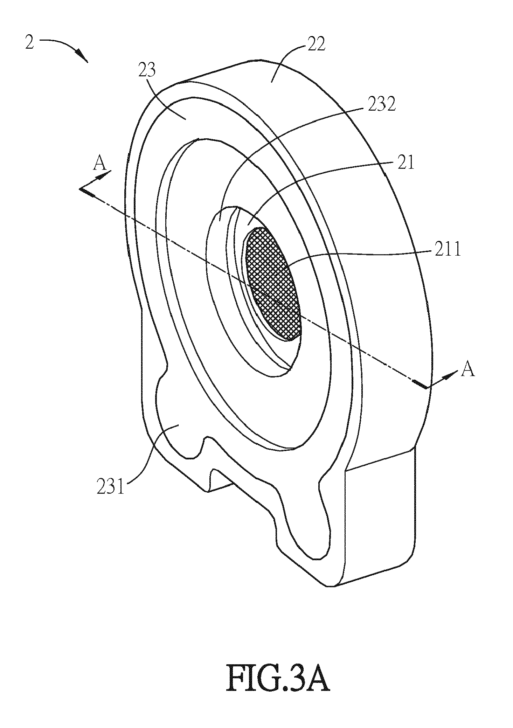

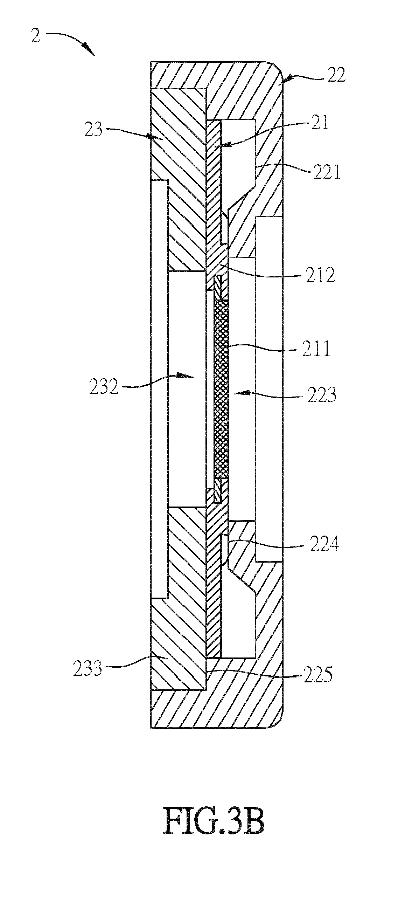

FIG. 3A is a schematic assembled diagram of the spraying module in FIG. 2, and FIG. 3B is a schematic sectional diagram of the spraying module taken along the line A-A in FIG. 3A. As shown in FIGS. 2, 3A, and 3B, in order to protect the piezoelectric element 212, the first covering element 22 is disposed on one side of the spraying element 21 and the second covering element 23 is disposed on the other side of the spraying element 21. In other words, the first and second covering elements 22 and 23 are disposed on the two opposite sides of sheet of the spraying element 21, respectively, and together cover the spraying element 21. In this embodiment, the first covering element 22 is substantially a circular structure and has a circular hollow portion 221 corresponding to the structure of the spraying element 21 to accommodate the spraying element 21. The first covering element 22 further includes two depressions 222, 222 which are extended from the hollow portion 221 and, for example but not limited to, stretch along two different directions, and therefore the first and second electrodes 213 and 214 can be disposed in the two depressions 222, respectively. The second covering element 23 is substantially a circular cover structure corresponding to the structures of the spraying element 21 and hollow portion 221, and is disposed on the side of the piezoelectric element 212 which opposite the first covering element 22, so that the spraying element 21 is clipped and wrapped between the first and second covering elements 22 and 23. In this embodiment, the second covering element 23 is also disposed in the hollow portion 221 to cover the spraying element 21. The second covering element 23 further includes two protrusions 231. When the second covering element 23 is disposed in the hollow portion 221, the two protrusions 231, 231 are disposed corresponding to the two depressions 222, 222 to cover the first and second electrodes 213 and 214, respectively.

In other embodiments, the spraying element can have other shapes. For example, the spraying element has a rectangular shape and the hollow portion of the first covering element and the second covering element have the corresponding rectangular structures. The spraying module and the elements thereof are not limited in shape in this invention, as long as they can match each other so that the first and second covering elements and can together cover the spraying element such that to wrap the spraying element to prevent liquid from contacting the spraying element.

Accordingly, the first and second covering elements 22 and 23 together cover the spraying element 21. Besides, in one embodiment, the first and second covering elements 22 and 23 are made by compliant material, which indicates soft material or the material with elasticity so as to show the compliance. The compliance is an antonym of the stiffness by definition herein. In an embodiment, the compliance indicates the extent to which a kind of material is compliant to the effect brought by an external force. The compliance possessed by the compliant material is just suitable and required in this invention. The compliant material is, for example but not limited to, silica gel, rubber, soft polymer or foam, and wherein the soft polymer can be PORON® (micro-cell polymer material, a commercial plastic material produced by ROGERS INOAC CORP.), EVA (ethylene-vinyl acetate), CR (chloroprene rubber), SBR (styrene-butadiene rubber), PU (polyurethane), PE (polyethylene) or the above-mentioned foam for example. Favorably, the first and second covering elements 22 and 23 include silica gel material. The fact that first and second covering elements 22 and 23 including the soft compliant material is not only mean they can protect the weaker piezoelectric element 212 from being damaged due to external force, but also can buffer the oscillation extent of the piezoelectric element 212 supplied with electricity, and can further provide a better sealing effect, too.

As shown in FIGS. 1B and 2, the first covering element 22 has an input opening 223. The mesh element 211 of the spraying element 21 has a mesh structure, i.e. a fine-holes structure, and the mesh structure of the mesh element 211 is disposed corresponding to the input opening 223. The second covering element 23 has an output opening 232, which is disposed corresponding to mesh structure of the mesh element 211. In this embodiment, the first covering element 22 further includes a flange 224 which is formed to surround the input opening 223. Also as shown in FIG. 3B, one side of the spraying element 21 is pressed by the flange 224 of the first covering element 22, and the other side of the spraying element 21 is pressed by the second covering element 23, and therefore the spraying element 21 can be stably clipped between the first and second covering elements 22 and 23. Besides, the oscillation range of the spraying element 21 in operation can be limited so that the piezoelectric element 212 can be prevented from being damaged due to overmuch oscillation extent.

In addition to the flange 224 of the first covering element 22, in this embodiment, the first covering element 22 further includes a first engaging structure 225, which is disposed on the inner wall of the first covering element 22 to substantially surround the hollow portion 221. Accordingly, when the spraying element 21 is disposed in the hollow portion 221, the portion of the spraying element 21 where near the center is pressed by the flange 224, and the portion of the spraying element 21 where near the edge is disposed on the inner side of the first engaging structure 225, as shown in FIG. 3B. The second covering element 23 further includes a second engaging structure 233, and the first engaging structure 225 matches the second engaging structure 233 in shape. In this embodiment, the first and second engaging structures 225 and 233 have the outer diameters matching each other to together cover the spraying element 21.

As shown in FIG. 1B, when the spraying element 21 is disposed in the accommodating space 11 of the main body 1, the opening 12 of the main body 1 is disposed corresponding to the input opening 223, and the output structure 3 is disposed on the outer side of the accommodating space 11 of the main body 1. The output structure 3 includes an output channel 31, and the output channel 31 is disposed corresponding to the mesh element 211 of the spraying module 21. In an embodiment, the output channel 31 communicates with the output opening 232 of the second covering element 23 so that the mesh structure of the mesh element 211 can directly correspond to the output channel 31. The liquid stored inside the main body 1 can be transmitted to the mesh structure of the mesh element 211 through the opening 12 and the input opening 223. After the electricity is supplied, the piezoelectric element 212 can convert electric energy into mechanical energy, and therefore the liquid can be atomized or sprayed into fine portions in an oscillation manner. In this embodiment, the liquid can be sprayed out through the mesh structure of the mesh element 211, and the diameter of the sprayed out liquid particle could be less than that of the mesh so that the atomization effect can be enhanced. The sprayed out liquid particles are transmitted to outside of the spraying device S through the output opening 232 and the output channel 31. Thereby, the user can aim the output channel 31 of the output structure 3 at the location to be sprayed, and thereby for example, to spray the cosmetic liquid on the skin for the beauty treatment or to spray the medicine over the mucous membrane of the respiratory tract of the throat.

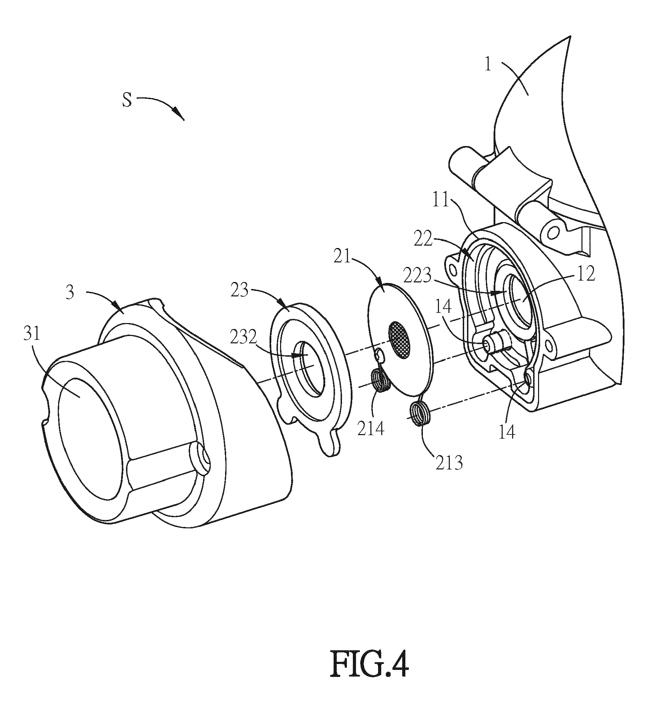

FIG. 4 is a schematic diagram showing the first covering element disposed at the accommodating space in FIG. 1B. As shown in FIGS. 2 and 4, the first covering element 22 of this embodiment includes two holes 226, 226 (one of the holes 226 is hidden due to the viewing angle of the figure) disposed at the corresponding two depressions 222, 222 respectively, so that the first and second electrodes 213 and 214 disposed in the two depressions 222, 222 are disposed corresponding to the two holes 226, 226, respectively. Besides, the pair of third electrodes 14, 14 of this embodiment are also disposed corresponding to the holes 226, 226 and exposed from the holes 226, 226 respectively so that the two third electrodes 14, 14 can be electrically connected to the corresponding first and second electrodes 213 and 214, respectively. Consequently, by the design of the holes 226 of the first covering element 22 and the second covering element 23 completely covering the whole structure of the spraying element 21, the first electrode 213, the second electrode 214 and the third electrodes 14 which can be prevented from being damped due to the contact with the moisture and the spraying device S can be prevented from the breakdown due to the short-circuit.

Furthermore, a praying module is further disclosed in this invention, including a spraying element, a first covering element and a second covering element. The spraying element includes a first electrode and a second electrode. The first covering element has two depressions. The first covering element is disposed on one side of the spraying element, and the first and second electrodes are disposed in the two depressions, respectively. The second covering element has two protrusions. The second covering element is disposed on the other side of the spraying element, and the two protrusions cover the first and second electrodes, respectively. The first and second covering elements are made by compliant material. The elements of the spraying module and the technical features thereof and the relation with other elements can be comprehended by referring to the above illustration and therefore are not described here for conciseness.

Summarily, in the spraying device and the spraying module of the invention, the first and second covering elements are disposed on two opposite sides of the spraying element and made by compliant material with softness and elasticity, so the spraying element, especially for the weaker or brittle element of the spraying element such as the piezoelectric element, can be protected by disposition and material property of the first and second covering elements. The first and second covering elements not only can protect the weaker or brittle piezoelectric element from being damaged due to the external force, but also can buffer the oscillation extent of the piezoelectric element supplied with electricity so that the piezoelectric element can be prevented from being damaged due to the overmuch oscillation extent. Besides, by use of the compliant material, the first and second covering elements can have better sealing effect so that the first, second and third electrodes can be prevented from being damped, and the lifespan of the spaying device and module can be thus extended.

Furthermore, the first covering element includes the depressions, and the second covering element correspondingly includes the protrusions to accommodate and cover the first and second electrodes. Therefore, the first, second and third electrodes can be enhanced due to sealed effect, and can be further prevented from the damp.

Although the invention has been described with reference to specific embodiments, this description is not meant to be construed in a limiting sense. Various modifications of the disclosed embodiments, as well as alternative embodiments, will be apparent to persons skilled in the art. It is, therefore, contemplated that the appended claims will cover all modifications that fall within the true scope of the invention.

## Claims

1. A spraying module, comprising:
a spraying element including a first electrode and a second electrode;
a first covering element having two depressions and disposed on one side of the spraying element, wherein the first electrode and the second electrode are disposed in the two depressions respectively; and
a second covering element having two protrusions and disposed on the other side of the spraying element, wherein the two protrusions cover the first electrode and the second electrode, respectively, and the first covering element and the second covering element are made by compliant material.

2. The spraying module as recited in claim 1, wherein the compliant material includes silica gel, rubber, soft polymer or foam.

3. The spraying module as recited in claim 2, wherein the soft polymer includes PORON® (micro-cell polymer material), EVA (ethylene-vinyl acetate), CR (chloroprene rubber), SBR (styrene-butadiene rubber), PU (polyurethane) or PE (polyethylene).

4. The spraying module as recited in claim 1, wherein the spraying element includes a piezoelectric element.

5. The spraying module as recited in claim 4, wherein the spraying element further includes a mesh element.

6. The spraying module as recited in claim 1, wherein the first covering element has an input opening, and the second covering element has an output opening.

7. The spraying module as recited in claim 1, wherein the first covering element includes a first engaging structure, the second covering element includes a second engaging structure, and the first engaging structure and the second engaging structure match each other.

8. The spraying module as recited in claim 1, wherein the first covering element has two holes disposed corresponding to the two depressions, respectively, and the first electrode and second electrode of the spraying element are disposed corresponding to the two holes, respectively.

9. A spraying device, comprising:
a main body including an accommodating space;
a spraying module disposed at the accommodating space and including:
a spraying element including a first electrode and a second electrode;
a first covering element having two depressions and disposed on one side of the spraying element, wherein the first electrode and the second electrode are disposed in the two depressions respectively; and
a second covering element having two protrusions and disposed on the other side of the spraying element, wherein the two protrusions cover the first electrode and the second electrode, respectively, and the first covering element and the second covering element are made by compliant material; and
an output structure having an output channel disposed corresponding to the spraying module.

10. The spraying device as recited in claim 9, wherein the spraying element further includes a mesh element.

11. The spraying device as recited in claim 9, wherein the first covering element has an input opening, and the second covering element has an output opening.

12. The spraying device as recited in claim 11, wherein the main body has an opening at the accommodating space to communicate with an inner portion and an outer portion of the main body, and the opening is disposed corresponding to the input opening.

13. The spraying device as recited in claim 9, wherein the first covering element includes a first engaging structure, the second covering element includes a second engaging structure, and the first engaging structure and the second engaging structure match each other.

14. The spraying device as recited in claim 9, wherein the first covering element has two holes disposed corresponding to the two depressions, respectively, and the first electrode and second electrode of the spraying element are disposed corresponding to the two holes, respectively.

15. The spraying device as recited in claim 14, wherein the main body further includes a pair of third electrodes which are disposed in the accommodating space and corresponding to the first electrode and the second electrode respectively.
